# EUROPEAN PATENT SPECIFICATION

(11) **EP 1 888 174 B1**
(45) Date of publication and mention of the grant of the patent: **22.02.2012**
(21) Application number: 06760236.7
(22) Date of filing: 19.05.2006
(51) Int. Cl.: A61K 31/12, A61K 31/00

(54) **USE OF CHALCONES FOR THE TREATMENT OF VIRAL DISORDERS**
VERWENDUNG VON CHALCONEN ZUR BEHANDLUNG VON VIRUSERKRANKUNGEN
UTILISATION DE CHALCONES POUR TRAITER DES TROUBLES D'ORDRE VIRAL

(30) Priority: 24.05.2005 US 137198
(43) Date of publication of application: 20.02.2008
(73) Proprietor: Johnson and Johnson Consumer Companies, Inc., Skillman, NJ 08558 (US); Galileo Pharmaceuticals, Inc., Santa Clara, CA 95054 (US)
(72) Inventor: BODDUPALLI, Sekhar, Palo Alto, CA 94306 (US); MAHMOOD, Khalid, Batavia, IL 60510 (US); SALIOU, Claude, Bernardsville, NJ 07924 (US)
(74) Representative: Fisher, Adrian John
(86) International application number: PCT/US2006/019626
(87) International publication number: WO 2006/127539

(56) References cited:
- WO-A-99/00114
- WO-A-2006/034219
- BUCKWOLD VICTOR E ET AL: "Antiviral activity of hop constituents against a series of DNA and RNA viruses." ANTIVIRAL RESEARCH. JAN 2004, vol. 61, no. 1, January 2004 (2004-01), pages 57-62, XP002398448 ISSN: 0166-3542
- ARTICO M ET AL: "GEOMETRICALLY AND CONFORMATIONALLY RESTRAINED CINNAMOYL COMPOUNDS AS INHIBITORS OF HIV-1 INTEGRASE: SYNTHESIS, BIOLOGICAL EVALUATION,AND MOLECULAR MODELING" JOURNAL OF MEDICINAL CHEMISTRY, AMERICAN CHEMICAL SOCIETY. WASHINGTON, US, vol. 4, no. 21, 8 October 1998 (1998-10-08), pages 3948-3960, XP000978720 ISSN: 0022-2623

## Description

### Background Information

The present invention relates to the use of chalcones for the treatment of viral disorders. In one embodiment, the invention relates to the treatment of viral lesions, for example, the treatment of cold sores resulting from the herpes simplex virus (HSV), with compositions containing chalcones.

Cold sores are often associated with an unpleasant stigmatism, however, the great number of individuals affected by the virus confirms it as a relevant virus in our society. Also referred to as fever blisters, cold sores are brought on by the herpes simplex virus which resides in the nerves of the cold sore sufferers. The herpes simplex virus is part of the herpes virus group and shares the distinct characteristic of the ability to lie dormant within the body, specifically in the nerve cells, for long periods of time, or for the lifetime of the individual.

Cold sores are contagious and reoccurring. The first outbreak often occurs 1 to 3 weeks after the virus has initially been contracted. The virus most often spread through contact with the open sores of an infected individual. However, the virus can also spread even in the absence of open sores or any symptoms. The sores initially appear as small, fluid-filled blisters on the skin.

Symptoms include, but are not limited to, fever, muscle aches, swollen glands, malaise, itching, inflammation, irritation, pain, swelling and burning. These symptoms are followed by an initial tingling sensation to the sufferer then followed by painful blisters. The usual duration of the sores can last from 2 to 3 weeks with the blisters scabbing and then eventually falling off the skin completely, generally without any scarring of the infected skin area.

Causes for the outbreaks include a weakening of the immune system from colds or other infections, the length of time the person infected has had the virus, exhaustion, emotional and physical stress, the menstrual cycle, immunosuppression, overexposure to wind and sunlight, and drug and heavy alcohol use.

The most common types of this virus are herpes simplex virus-1 (HSV-1) and herpes simplex virus-2 (HSV-2). The main difference between the two viruses is where they set up their dormancy site in the body. HSV-1 usually lies dormant in the trigeminal ganglion, the nerve cells located around the ear, whereas HSV-2 usually lies dormant in the sacral ganglion, the nerve cells located at the base of the spine.

HSV-1 is responsible for the formation of cold sores formed around the lips and less infrequently, the chin, nostrils, fingers and the gums and roof of the mouth of an infected individual. Extremely uncommon, although possible and less known to the public, is the formation of cold sores in the genital area from HSV-1 sufferers. Dismissed by the public as more socially acceptable and generally more of an inconvenience than a health risk, HSV-1 can cause serious dangers to an infected individual. HSV-1' can spread to the eye causing ocular herpes, which can cause blindness. HSV-1 also has the ability to spread to the brain, causing herpes encephalitis, which can lead to death.

The most infamous of the herpes simplex viruses, HSV-2 is responsible for cold sores in the genital area. Approximately 1 in 4 individuals are believed to be infected with HSV-2. HSV-2 can also be spread to the eye and brain, as discussed above. Not only is HSV-2 an uncomfortable and often painful physical affliction, it can cause emotional and psychological suffering to the affected individual.

Herpes simplex virus-3 (HSV-3), also known as the varicella-zoster virus, causes chickenpox and later, shingles. Varicella is the primary infection that causes chickenpox. Chickenpox initially appears as small, red bumps on the abdomen, chest or face, later forming into blisters that eventually scab and fall off the body. Although symptoms do not show until about two days after exposure to the virus, the symptoms can last from five to ten days and include a red itchy rash, fever and headache.

Highly contagious, chickenpox is spread by an infected individual, most often through sneezing, coughing and breathing. It is then inhaled in the newly infected individual's lungs, passing into the bloodstream. Entering the nerve cells, the virus lays dormant for years, even a lifetime, possibly reappearing as herpes zoster or shingles. Shingles is thought to be only contracted from an individual with chickenpox, never from someone with shingles. The initial symptoms of shingles usually include a tingling feeling, itchiness, numbness, or stabbing pain in or under the skin. Shingles generally affects one side of the body, characterized by an outbreak of severely painful and itchy blisters. Postherpetic neuralgia can occur as a painful aftereffect of shingles. Treatments for postherpetic neuralgia include steroids, antiviral drugs, antidepressants, anticonvulsants, and topical agents.

The treatment of cold sores with certain chalcones isolated from the Stem Bark of Millettia leucantha has been described in Phrutivorapongkpul et al, Chem. Pharm. Bull 51 (2) 187-190 (2003). US Patent No. 4,327,088 discloses phosphonoxy-substituted acrylophenones as useful in treating infections caused by human rhinoviruses, enteroviruses and influenzaviruses.

Antivirals such as acyclovir, valcyclovir or farcyclovir can be used to treat oral and genital HSV-1 and HSV-2, as well as shingles and chickenpox resulting from HSV-3. These antivirals reduce the amount of time that it takes for the blisters to heal and can be taken orally on a regular basis in order to prevent reoccurrences. However, these existing drugs may cause side effects such as, for example, nausea, vomiting, diarrhea, headache, dizziness, and/or rashes, and some users may experience disorientation, hallucinations, delirium and tremors. Abnormal renal function can also occur as a result of acyclovir, valcyclovir or farmcyclovir administration. Patients with preexisting renal dysfunction or dehydration, or with hepatic dysfunction are advised to use it with caution. Abnormal renal function can also occur due to drug interactions with nephrotoxic drugs, some pain medicines, and cyclosporine.

Therefore, new effective and safe compositions to treat viral lesions, such as cold sores, by minimizing and/or eliminating the number and/or severity of lesions, are desirable.

### SUMMARY OF THE INVENTION

The present invention relates to compositions containing chalcones for specific uses and compounds for use in methods for using such chalcones as defined in the claims.

In one embodiment, the present invention relates to compounds for use in a method for the treatment of a cold sore including administering to a subject in need of such treatment a composition containing compounds of Formula IA: 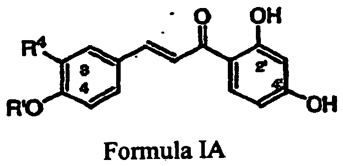 wherein,
R⁴ is hydrogen or -OR'; and
R' is hydrogen or C₁-C₆-alkyl;
including single stereoisomers, mixtures of stereoisomers, and pharmaceutically acceptable salts thereof.

In some embodiments, the viral lesions result from a virus selected from a herpes virus (e.g., HSV-1, HSV-2, or HSV-3). In other embodiments the viral lesions are cold sores.

In some embodiments, the method of administration is topical, such as topically applying the compound to the lesion. In other embodiments the method of administration is oral.

In another aspect, the disclosure relates to compounds for use in a method for the treatment of one or more symptoms associated with viral infections in a subject suffering from a virus, such as a herpes simplex virus, with a composition containing a compound of Formula IA. In some embodiments, the symptoms include fever, muscle aches, swollen glands, malaise, itching, inflammation, irritation, pain, swelling and burning.

In some embodiments, the invention relates to compositions for the treatment of viral lesions including at least one of the following compounds: 2',4' dihydroxy 2,3-dimethoxychalcone; 2'-hydroxy-3,4-dimethoxychalcone; 2'-hydroxy-4,4'-dimethoxychalcone; 6'-hydroxy-2,2',3',4,4'-pentamethoxychalcone; 2'-hydroxy-2,2',4-dimethoxychalcone; 2'-hydroxy-2,3, 5-trimethoxychalcone; 2'-hydroxy-2,4,4'-trimethoxychalcone; 2'-hydroxy-2,4,5'-trimethoxychalcone; 2'-hydroxy-3,4,5'-trimethoxychalcone; 2,2',4'-trihydroxychalcone; 2',4,4'-trihydroxychalcone; 2',3',4'-trihydroxychalcone; 2',4,5'-trimethoxychalcone; 2',4,5'-trihydroxychalcone; 2',3,4,5'-tetramethoxychalcone; 2'-hydroxy-2,4,5'-trimethoxychalcone; 2'4'-dihydroxy-3,4-dimethoxychalcone; 2',4'-dihydroxy-2,3-dimethoxychalcohe; 2',4;4',6'-tetramethoxychalcone; 2',3,4,4',6'-pentamethoxychalcone; 4-hydroxy-2',4',6'-trimethoxychalcone; 2',4,4',6'-tetramethoxychalcone; 2'-hydroxy-3,3',4,5-tetramethoxychalcone;2,4-dihydroxy-2',4',6'-tetramethoxychalcone; 3-hydroxy-2',4,4',6'-tetramethoxychalcone; 4'-hydroxy-2'-methyl-3,4,5-trimethoxychalcone;4'-hydroxy-2'-methyl-4-methoxychalcone; 2',4,4'-trihydroxy-3-methoxychalcone; 5-geranyl-2,2'4,4'-tetrahydroxychalcone; 3'4,4'-trihydroxy-2-methoxy-3-prenylchalcone; 4,4'-dihydroxy-3,5-diprenylchalcone; 2',4,4'-trihydroxy-3-prenylchalcone; 2',4,4'-trihydroxy-3, 5'-diprenylchalcone; 2',4,4' -trihydroxy-3,3',5-triprenylchalcone; 2',3,3',4,4'-pentahydroxy-3'-prenylchalcone;2,2',4,-trihydroxy-3-prenylchalcone; 2',3,4,4',6'-pentahydroxy-2,5-diprenylchalcone; 4,4',6'-trihydroxy-3,3'-diprenylchalcone; 3,4,4'-trihydroxy-2-methoxychalcone; 2',6'-dihydroxy-4-methoxychalcone; 2'-hydroxy-3',4'-dimethoxychalcone; 2'-hydroxy-2,4-dimethoxychalcone; 2'-hydroxy-2,4'-dimethoxychalcone; 2'4,4'-trihydroxy-6-methoxy-3-prenylchalcone; 3'4,4'-trihydroxy-2-methoxy-3-prenylchalcone; 2'-hydroxy-2,4'-dimethoxychalcone, and 5-bromo-4-hydroxy-3,4'-dimethoxychalcone.

The present invention relates to a compound of Formula IA for use in a method for the treatment of viral infections including administering to a subject in need of such treatment a composition containing compounds of Formula IA: 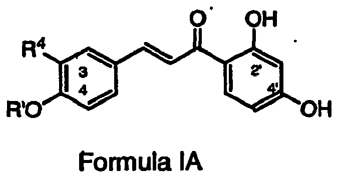 wherein,
R⁴ is hydrogen or -OR'; and
R' is hydrogen or C₁-C₆-alkyl;
including single stereoisomers, mixtures of stereoisomers, and pharmaceutically acceptable salts thereof.

In some embodiments the viral lesions are cold sores.

In another aspect, the disclosure relates to a method for the treatment of one or more symptoms associated with viral infections, in a subject suffering from a virus, such as a herpes simplex virus, with a composition containing a compound of
Formula IA. In some embodiments, the symptoms include fever, muscle aches, swollen glands, malaise, itching, inflammation, irritation, pain, swelling and burning.

In some embodiments the compositions contain a compound selected from 2",4'-dihydroxy-3,4-dimethoxychalcone, 2'4'-dihydroxy-4-methoxychalcone, 2',3,4,4'-tetrahydroxychalcone, 2',4',4-trihydroxychalcone, 2',4,4'-trihydroxy-3-methoxychalcone, and 2',3,4'-trihydroxy-4-methoxychalcone including single stereoisomers, mixtures of stereoisomers, and pharmaceutically acceptable salts thereof.

In some embodiments, the compositions contain 2,4'-dihydroxy-3,4-dimethoxychalcone with the following structure: 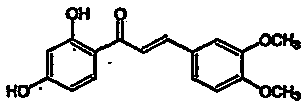 including single stereoisomers, mixtures of stereoisomers, and pharmaceutically acceptable salts thereof.

In other embodiments, the compositions contain compound 2',3,4,4'-tetrahydroxychalcone with the following structure: 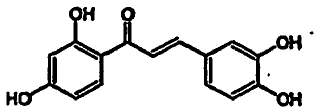 including single stereoisomers, mixtures of stereoisomers, and pharmaceutically acceptable salts thereof.

In other embodiments, the compositions contain compound 2',4'-dihydroxy-4-methoxychalcone with the following structure: 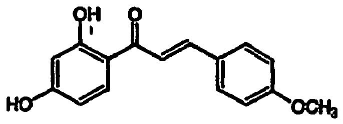 including single stereoisomers, mixtures of stereoisomers, and pharmaceutically acceptable salts thereof.

In other embodiments, the compositions contain compound 2',4',4-trihydroxychalcone with the following structure 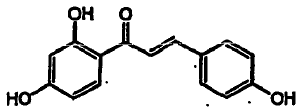 including single stereoisomers, mixtures of stereoisomers, and pharmaceutically acceptable salts thereof.

In other embodiments the compositions contain compound 2',4,4'-trihydroxy-3-methoxychalcone 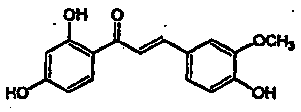 including single stereoisomers, mixtures of stereoisomers, and pharmaceutically acceptable salts thereof.

In other embodiments the compositions contain compound 2',3,4'-trihydroxy-4-methoxychalcone: 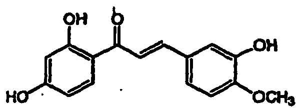 including single stereoisomers, mixtures of stereoisomers, and pharmaceutically acceptable salts thereof.

In some embodiments, the viral lesions result from a virus selected from herpes simplex virus-1, herpes simplex virus-2, and herpes simplex virus- 3.

In another embodiment, the invention relates to a composition for use in a method for controlling viral growth and replication resulting from herpes simplex virus, said method including administering to the subject in need of such treatment, a composition containing a compound of Formula IA, such as 2',4' -dihydroxy-3,4-dimethoxychalcone, 2',4'-dihydroxy-4-methoxychalcone, 2',3,4,4'-tetrahydroxychalcone, 2',4',4-trihydroxychalcone, 2',4,4'-trihydroxy-3-methoxychalcone, and 2',3,4'-trihydroxy-4-methoxychalcone, including single stereoisomers, mixtures of stereoisomers, and pharmaceutically acceptable salts thereof.

In another embodiments, the invention relates to the composition being included in a topical formulation. In another embodiment, the composition is administered orally.

In other embodiments, the invention relates to the composition for use in a method of treatment with a composition that further contains at least one of the following: (i) a skin protectant active ingredient selected from allantoin, aluminum hydroxide gel, calamine, cocoa butter, cod liver oil, colloidal oatmeal, dimethicone, glycerin, hard fat, kaolin, lanolin, mineral oil, petrolatum, sodium bicarbonate, topical starch, white petrolatum, zinc acetate, and/or zinc oxide; (ii) an external, anesthetic or antipruritic ingredient selected from benzocaine, butamaben picrate, dibucaine, dibucaine hydrochloride, dimethiosoquin hydrochloride, dyclonine hydrochloride, lidocaine, lidocaine, hydrochloride, pramoxine hydrochloride, tetracaine, tetracaine hydrochloride, benzyl alcohol, camphor, camphorated metacresol, juniper tar, menthol, phenol, phenolate sodium resorcinol, tripelennamine hydrochloride, aspirin, hydrocortisone, hydrocortisone acetate and/or diphenydramine hydrochloride; and/or (iii) an other ingredient selected from allyl isothiocyanate, ammonia solution, aspirin, bismuth sodium tartrate, capsaicin, capsicum oleoresin, chloral hydrate, chlorobutanol, cyclomethycaine sulfate, eucalyptus, eugenol, glycol salicylate, hexylresorcinol, histamine dihydrochloride, metapyriline hydrochloride, methyl nicotinate, methyl salicylate, pectin, salicylamide, tannic acid, thymol, trolamine salicylate, turpentine oil, zinc sulfate, aluminum acetate, aluminum sulfate, sucrose stereate, sucrose distereate, and/or witch hazel.

In other embodiments, the invention relates to the composition for use in a method of treatment with a composition that further contains one or more agents selected from the group consisting of anti-microbial agents, other antiviral agents, antifungal agents, antioxidants, buffering agents, sunscreens, cosmetic agents, fragrances, lubricants, moisturizers, drying agents, and thickening agents.

### DETAILED DESCRIPTION OF THE INVENTION

### Definitions

As used in the present specification, the following words and phrases are generally intended to have the meanings as set forth below, except to the extent that the context in which they are used indicates otherwise.

The term "astringent active ingredients" includes but is not limited to, aluminum acetate, aluminum sulfate, and witch hazel.

The term "acyl" refers to the groups -C(O)-H, -C(O)-(alkyl), -C(O)-(cycloalkyl), -C(O)-(alkenyl).

The term "alkenyl" refers to a monoradical branched or unbranched, unsaturated or polyunsaturated hydrocarbon chain, having from about 2 to 10 carbon atoms. This term is exemplified by groups such as ethenyl, but-2-enyl, 3-methyl-but-2-enyl (also referred to as "prenyl", octa-2,6-dienyl, 3,7-dimethyl-octa-2,6-dienyl (also referred to as "geranyl"), and the like.

The term "alkyl" refers to a monoradical branched or unbranched saturated hydrocarbon chain. This term is exemplified by groups such as methyl and includes ethyl, propyl, butyl, t-butyl, pentyl, and the like.

The term "alkoxycarbonyl" means a radical "-COOR'", wherein R' is an alkyl group as defined hereih. Examples of alkoxycarbonyl radicals include, but are not limited to, methoxycarbonyl, ethoxycarbonyl, and the like.

The term "carboxy" refers to the moiety "-COOH."

The term "cosmetics" includes make-up, foundation, and skin care products. The term "make-up" refers to products that leave color on the face, including foundations, i.e., concealers, lip balms, lipsticks and so forth. The term "foundation" refers to liquid, creme, mousse, compact, concealer, or like products that even out the overall coloring of the skin. Foundation is typically manufactured to work better over moisturized and/or oiled skin. The term "skin care products" refers to products used to treat or otherwise care for, moisturize, improve, or clean the skin. The term "cosmetics" may also include other safe skin protectant drug products for over-the-counter human use as defined in the code of federal regulations such as 21 CFR 347 and 21 CFR 348.

The term "compound of Formula I" or "compound of Formula IA" is intended to encompass the derivatives of the invention as disclosed, stereoisomers, mixtures of stereoisomers and/or the pharmaceutically acceptable salts of such compounds. In addition, the compounds employed in this invention include the individual stereochemical isomers and mixtures of isomers. For the sake of brevity, except where specifically indicated to the contrary (e.g., by designation of a single salt, isomer or mixture), the term should be understood to include single stereoisomers, mixtures of stereoisomers, pharmaceutically acceptable salts, and prodrugs thereof.

The term "effective, amount" refers to that amount of a compound of the present invention that is sufficient to effect treatment, as defined herein, when administered to a mammal in need of such treatment. The effective amount will vary depending upon the subject and disease condition being treated, the weight and age of the subject, the severity of the disease condition, the particular compound chosen, the dosing regimen to be followed, and the like, all of which can readily be determined by one of ordinary skill in the art.

The term "external analgesic, anesthetic, and antipruritic active ingredients" includes, but is not limited to, benzocaine, butamaben picrate, dibucaine, dibucaine hydrochloride, dimethiosoquin hydrochloride, dyclonine hydrochloride, lidocaine, lidocaine hydrochloride, pramoxine hydrochloride, tetracaine, tetracaine hydrochloride, benzyl alcohol, camphor, camphorated metacresol, juniper tar, menthol, phenol, phenolate sodium resorcinol, tripelennamine hydrochloride, aspirin, hydrocortisone, hydrocortisone acetate and diphenydramine hydrochloride.

The term "inflammation" refers to the localized protective response elicited by the destruction of tissues. It is characterized by signs of pain, heat, redness, and/or swelling.

The term "halogen" refers to fluoro, chloro, bromo, and iodo.

The term "glycosyl" refers to a 5 or 6 carbon sugar derivate. Examples of glycosyl include but are not limited to allosyl, altrosyl, glucosyl, mannosyl, gulosyl, idosyl, galactosyl, talosyl, arabinosyl, xylosyl, lyxosyl, rhamnosyl, ribosyl, deoxyfuranosyl, deoxypyranosyl, and deoxyribosyl. The glycose may be azide substituted, or O-acetylated, O-alkylated, O-sylilated, O-stannylated or O-stannylidenated.

The term "pharmaceutically acceptable" refers to those compounds, materials, compositions, and/or dosage forms which are, within the scope of sound medical judgment, suitable for contact with the tissues of human beings and animals without excessive toxicity, irritation, allergic response, or other problem complications commensurate with a reasonable benefit/risk ratio.

The term "pharmaceutically acceptable salts" refers to salts which retain the biological effectiveness and properties of the compounds of this invention and which are not biologically or otherwise undesirable, In some cases, the compounds of this invention are capable of forming salts by virtue of the presence of phenolic, and/or carboxyl groups. Pharmaceutically acceptable base addition salts can be prepared from inorganic and organic bases. Salts derived from inorganic bases, include by way of example only, sodium, potassium, lithium, ammonium, calcium and magnesium salts. Salts derived from organic bases include by way of example only salts of primary, secondary and tertiary amines.

The term "prodrug" refers to an inactive form of a compound which must be metabolized *in vivo,* e.g., by biological fluids or enzymes, and/or by a subject after administration into an active form of the compound in order to produce the desired pharmacological effect. The prodrug can be metabolized before absorption, during absorption, after absorption, or at a specific site. Prodrug forms of compounds may be utilized, for example, to improve bioavailability; improve subject acceptability such as masking or reducing unpleasant characteristics such as a bitter tastes odor, or gastrointestinal irritability; alter solubility; provide for prolonged or sustained release or delivery; improve ease of formulation; and/or provide site-specific delivery of the compound. Reference to a compound herein includes prodrug forms of a compound.

The term "stereoisomers" refers to compounds that have identical molecular formulae but that differ in the arrangement of their atoms in space. Configurations of stereoisomers that owe their existence to hindered rotation about double bonds are differentiated by their prefixes cis and trans, (Z and E), which indicate that the groups are on the same side (cis or Z) or on opposite sides (trans or E) of the double bond in the molecule according to the Cahn-Ingold-Prelog rules.

The term "skin care products" may include, but is not limited to, skin protectant active ingredients, astringent active ingredients, external analgesic, anesthetic and antipruritic active ingredients as published in 21 CFR 347.10, 347.12 and 348.10, and other ingredients as published in 55 FR 3370, or mixtures thererof.

The term "skin protectant active ingredients" include but are not limited to allantoin, aluminum,hydroxide gel, calamine, cocoa butter, cod liver oil, colloidal oatmeal, dimethicone, glycerin, hard fat, kaolin, lanolin, mineral oil, petrolatum, sodium bicarbonate, topical starch, white petrolatum, zinc acetate, and zinc oxide. Skin protectant active ingredients may also include sunscreen agents.

The term "sunscreen" may include, but is not limited to, organic or inorganic sunscreens, sun blocks titanium oxide and zinc oxide, and skin protectants and/or mixtures thereof. Sunscreen products providing a minimum SPF value of not less than 2, include, but are not limited to, aminobenzoic acid (PABA); avobenzone, cinoxate, dioxybenzone, homosalate, menthyl anthranilate, methoxycinnamate, octocrylene, octyl methoxycinnamate, octyl salicylate, oxybenzone, padimate O, phenylbenzimidazole sulfonic acid, sulisobenzone, titanium dioxide, trolamine salicylate, titanium oxide, and zinc oxide.

The term "topical application" means directly laying on or spreading on outer skin using, e.g., by use of the hands or an applicator such as a wipe, puff, roller, or spray. As used herein, "topical carrier" means one or more compatible solid or liquid filler diluents that are suitable for topical administration to a mammal. Examples of topical carriers include, but are not limited to, water, waxes, oils, emollients, emulsifiers, thickening agents, gelling agents, and mixtures thereof.

The term "treatment" or "treating" includes (i) inhibiting the disease or disorder, that is, arresting or suppressing the development of clinical symptoms of the disease or disorder; and/or (ii) relieving the disease or disorder, that is, causing the regression or cure of clinical symptoms of the disease or disorder. Examples include, but are not limited to, supressing the reoccurence or severity of symptoms of viral infections, such as lesions.

The term "viral lesions" refers to all lesions which have been affected by viral disorders such as all herpes, including, but not restricted to, cold sores, genital herpes, shingles, chicken pox, forms of zoster, and other disorders of viral nature. This term is not restricted to orofacial lesions and includes manifestations on all parts of the body.

### Compounds of the Invention

Certain compounds of Formula IA may be synthetic materials or may be obtained as extracts from natural sources. Some illustrative plant genuses where these compounds may be sourced from are primula, trifolium, bidens, and/or coreopsis.

Synthetic routes for the production of compounds of the present invention have been described in the literature, for example in Bioorganic & Chem. Letters (2004) 14, 3913-3916, Indian Journal of Chemistry (2003) 42B, 202-205, or Indian Journal of Chemistry (1988) 27B, 67.

These compounds may also be available from commercial sources such as Indofine Chemical Company, Inc. (Somerville, NJ) and Sigma-Aldrich (Milwaukee; WI).

### Utility, Testing and Administration

### General Utility

Compounds, compositions/formulations of the present invention are useful in treating or managing viral diseases, arid/or symptoms thereof. Viral diseases can include, but are not limited to: molluscum contagiosum; human T-cell lymphotropic virus (HTLV); human immuno-deficiency virus (HIV); acquired immuno-deficiency virus (AIDS); human papillomavirus; herpesvirus; herpes; viral dysentery; arenavirus; coronavirus; enterovirus; common cold; flu; measles; rubella; chicken pox; mumps; polio; rabies; mononucleosis; ebola; respiratory syncytial virus; dengue fever; yellow fever; lassa fever; bunyavirus; filovirus; flavivirus; hantavirus; rotavirus; West Nile fever; arbovirus; parainfluenza; smallpox; Epstein-Barr virus; cytomegalovirus; viral gastroenteritis; acute appendicitis; hepatitis (A-E; X); cold sores; meningitis; encephalitis; shingles; pneumonia; Rift Valley fever; hendra fever; roseola; sandfly fever; severe acute respiratory syndrome (SARS); warts; cat scratch disease; slap-cheek syndrome; orf; hand, foot and mouth disease; and pityriasis rosea.

It is an objective of this invention to control viral diseases such as herpes simplex viruses (HSV-1, HSV-2, or HSV-3). It is another objective of this invention to improve the symptoms associated with viral infections, such as but not limited to, fever, muscle aches, swollen glands, malaise, itching, inflammation, irritation, pain, swelling and burning.

It is an objective of this invention to provide improved compositions for the treatment or management of HSV-1 and HSV-2 during the active phrase of the virus. It is another objective of this invention to provide compositions that would help clear up and/or reduce the number of cold sores resulting from HSV-1 and HSV-2. It is another objective of this invention to provide compositions to help prevent the outbreak of new cold sores resulting from HSV-1 and HSV-2. It is another objective of this invention to reduce the healing time of the cold sores.

It is another objective of this invention to control viral growth and/or replication resulting from HSV-1 and HSV-2.

It is another objective of this invention to provide compositions and ingredients for compositions that can be used in combination with conventional viral lesion medications to reduce their appearance. It is also an objective of the invention to provide compositions for use in methods for using compositions of the invention with conventional viral lesion treatments for new combination therapies that maximize viral lesion management. It is a corresponding objective to alleviate the negative social and psychological impacts frequently suffered by persons afflicted with HSV-1 and HSV-2.

It is an objective of this invention to provide improved compositions for the treatment and management of HSV-3 during the active phase of the virus. It is another objective of this invention to provide compositions that would help clear up and/or reduce the number of chickenpox resulting from HSV-3.

It is another objective of this invention to provide compositions and ingredients for compositions that can be used in combination with conventional chickenpox medications to reduce their appearance and/or reduce associated inflammation and irritation, including itching. It is also another objective of the present invention to provide methods for using compositions of the invention with conventional chickenpox treatments to provide new combination therapies that maximize chickenpox maintenance.

It is another objective of this invention to provide compositions that would help clear up and/or reduce the number of shingles resulting from HSV-3. It is another objective of this invention to provide compositions and ingredients for compositions that can be used in combination with conventional shingles medications to reduce the appearance of and/or reduce associated symptoms ranging from mild itching to severe and intense pain. It is another objective of the invention to provide compositions for use in methods for using compositions of the invention with conventional shingles treatments to provide new combination therapies that maximize shingles maintenance.

### Testing

This section describes how compositions incorporating compounds of the present invention are'selected.

*In vitro* evaluation of anti-viral activity can be determined by plaque reduction as reported in J. Nat. Prod. (1990) 53, 340-344; or as described in Example 1. To pre-grown Vero cells (ATCC CCL-81 is added a virus suspension (ATCC VR-260) mixed with complete medium containing various concentrations of the test compound and the mixture is incubated until maximum cytopathic effect (CPE) is observed in the untreated virus control culture. The CPE inhibition is determined by adding a dye (MTS, (3-[4,5-dimethylthiazol-2-yl-5]-[3-carboxymethyoxyphenyl]-2-[4-sulfophenyl]-2H tetrazolium)) uptake procedure (Promega's Cell Titer Aqueous One Solution). This method measures cell viability and is based on the reduction of the tetrazolium-based MTS by mitochondrial enzymes of viable host ceiis to MTS formazan. The purple color of the MTS formazan is then measured spectrophotometrically. The optical density (OD) value of each culture is a function of the amount of formazan produced which is proportional to the number of viable cells. Compounds of the present invention showed superior anti-viral activity as described in Table I.

- *In vitro* cell-based assays for inflammation are well known in the art, for example, E-selectin (also named Endothelial Leukocyte Adhesion Molecule or ELAM) or interleukin-6 (IL-6). The ELAM assay measures *in vitro* activity of the test compounds in reducing expression of ELAM in activated endothelial cells. Briefly, endothelial cells are created by adding known activators such as lipopolysaccharides, TN F or IL-1β, alone or in some combination. Activated cells produce ELAM, which can be measured using, for example, an E-selectin monoclonal antibody-based ELISA assay, as described in Examples.

The IL-6 assay measures the release of IL-6 from a rat macrophage cell line following an inflammatory challenge with LPS and the ability of test articles to inhibit this activation and release. IL-6 is measured by a rat IL-6 ELISA and cell toxicity is determined using Cell Tracker.

*In vivo* evaluation of anti-inflammatory activity can be determined by well characterized assays measuring Carrageenan-Induced Paw Edema and by Mouse Ear Inflammatory Response to Topical Arachidonic Acid (Gabor, M., Mouse Ear Inflammation Models and their Pharmacological Applications, 2000). Carrageenan-Induced Paw Edema is a model of inflammation, which causes time-dependent edema formation following carrageenan administration into the intraplantar surface of a rat paw. The application of arachidonic acid (AA) to the ears of mice produces immediate vasodilation and erythema, followed by the abrupt development of edema, which is maximal at 40 to 60 min. The onset of edema coincides with the extravasations of protein and leukocytes. After one hour, the edema wanes rapidly and the inflammatory cells leave the tissue so that at 6 hours the ears have, returned to near normal. This assay measures a test compound's ability to treat these inflammatory processes via systemic route of administration.

Cytotoxic activity can be evaluated in cell culture using high glutamate induced oxidative stress (HGOS) in mouse dopaminergic cell lines. The cytotoxic effect of glutamate is not due to excitotoxicity, as this cell line is devoid of inotropic glutamate receptors. Rather, the glutamate-induced toxicity of dopaminergic cells is associated with an inhibition of cystine transport which subsequent leads to depletion of intracellular glutathione (GSH) levels (Murphy T. H., et al., Neuron 2, 1547 -1558, 1989), activation of neuronal 12-lipoxygenase (Li, Y. et al., Neuron 19,453 -463, 1997), increased ROS production (Tan S. et al., J. Cell Biol. 141, 1423 -1432,-1998) and elevated intracellular Ca²⁺ (Li, Y. *et al.,* see *supra*). Some molecules were measured for their ability to protect cells against glutamate-induced stress and the assay is detailed in Examples.

### Administration

In one embodiment, the compounds of the invention are administered at a pharmaceutically effective amount, e.g., a dosage sufficient to provide treatment for the disease states previously described. Administration of the compounds of the invention or the pharmaceutically acceptable salts thereof can be via any of the accepted modes of administration for agents that serve similar utilities.

In employing the compounds of this invention for treatment of the above conditions, any pharmaceutically acceptable mode of administration can be used. The compounds of the invention can be administered either alone or in combination with other pharmaceutically acceptable excipients, including solid, semi-solid, liquid, or aerosol dosage forms, such as, for example, tablets, capsules, powders, granules, cachets, liquids, suspensions, solutions, suppositories, aerosols, or the like. The compounds of the present invention can also be administered in sustained or controlled release dosage forms, including depot injections, osmotic pumps, pills, transdermal (including electrotransport) patches, and the like, for the prolonged administration of the compound at a predetermined rate, i.e., in unit dosage forms suitable for single administration of precise dosages. The compositions will typically include a conventional pharmaceutical carrier or excipient and a compound of the present invention or a pharmaceutically acceptable salt thereof. In addition, these compositions may include other medicinal agents, pharmaceutical agents, carriers, adjuvants, and the like, including, but not limited to, permeability enhancers and slow release formulations.

The active compound may be effective over a wide dosage range and is generally administered in a pharmaceutically effective amount, as described above. It will be understood, however, that the amount of the compound actually administered will, in the case of a pharmaceutical, be determined by a physician, in the light of the relevant circumstances, including the condition to be treated, the chosen route of administration, the actual compound administered, the age, weight, and response of the individual patient, the severity of the patient's syndrome, and the like. In the case of a cosmetic or over-the-counter skin care preparation, the actual amount of compound desired to be administered by the consumer will be recommended by the manufacturer, based on the manufacturer's test results, which may, in whole or in part, be determined on the basis of one or more of the *in vitro* and/or *in vivo* tests described herein,

The compositions of the present invention are suitable for treating viral lesions and their symptoms such as, but not limited to, fever, muscle aches, swollen glands, malaise, itching, inflammation, irritation, pain, swelling, and burning. The composition can be topically applied to the skin. In one embodiment, the topical composition contains the compound in an amount from about 0.001% to about 20%, by weight of the composition, such as from about 0.01 % to about 10%, by weight of the composition, such as from about 0.1% to about 5%, by weight of the composition. Compositions of the present invention can be topically applied by spraying, dabbing, dusting, swabbing, sponging, brushing, pouring, dispensing, covering, and heavily coating the affected area.

Compounds of the invention may be employed in skin care applications where treatment or amelioration of viral lesions is desirable. For example, compounds and compositions of the invention may be incorporated into leave-on preparations: wipes;, towelettes; swabs; lotions; salves; gels; creams; lotions; oils; ointments; pastes; balms; tinctures; emulsions; colloidal suspensions; lipsticks; and stick compositions.

Compositions useful for topical administration of the compositions of the present invention formulated as solutions typically include a pharmaceutically-acceptable aqueous or organic solvent. The term pharmaceutically-acceptable organic solvent refers to a solvent which is capable of having a composition of the present invention dispersed or dissolved therein, and of possessing acceptable safety properties (e.g., irritation and sensitization characteristics). Examples of suitable organic solvents include: propylene glycol; polyethylene glycol (200-600); polypropylene glycol (425-2025); glycerol; 1,2,4-butanetriol; sorbitol esters; 1,2,6-hexanetriol; ethanol; isopropanol; butanetriol; sorbitol esters; 1,2,6-hexanetriol; ethanol; isopropanol; butanediol; and mixtures thereof.

Topical formulations of the present invention typically contain the novel composition of the invention and optionally, a polar solvent. Solvents suitable for use in the formulations of the present invention include any polar solvent capable of dissolving the novel composition of the invention. Suitable polar solvents include: water, alcohols (such as ethanol, propyl alcohol, isopropyl alcohol, hexanol, and benzyl alcohol); polyols (such as propylene glycol; polypropylene glycol, butylene glycol, hexylene glycol, sorbitol, and glycerin); and panthenol dissolved in glycerin, flavor oils and mixtures thereof. Mixtures of these solvents can also be used. Exemplary polar solvents are polyhydric alcohols and water, such as but not limited to, glycerin, panthenol in glycerin, glycols such as propylene glycol and butylene glycol, polyethylene glycols, water and mixtures thereof.

An emollient may also be added to the topical compositions of the present invention. The emollient component can include fats, oils, fatty alcohols, fatty acids and esters which aid application and adhesion, yield gloss, and most importantly, provide occlusive moisturization. Suitable emollients for use are isostearic acid derivatives, isopropyl palmitate, lanolin oil, diisopropyl dimerate, maleated soybean oil, octyl palmitate, isopropyl isostearate, cetyl lactate, cetyl ricinoleate, tocopheryl acetate, acetylated lanolin alcohol, cetyl acetate, phenyl trimithicone, glyceryl oleate, tocopheryl linoleate, wheat germ glycerides, arachidyl propionate, myristyl lactate, decyl oleate, propylene glycol ricinoleate, isopropyl lanolate, pentaerythrityl tetrastearate, neopentylglycol dicaprylate/dicaprate, hydrogenated coco-glycerides, isononyl isononanoate, isotridecyl isononanoate, myristal myristate, triisocetyl citrate, cetyl alcohol, octyl dodecanol, oleyl alcohol, panthenol, lanolin alcohol, linoleic acid, linolenic acid, sucrose esters of fatty acids, octyl hydroxystearate and mixtures thereof. Examples of other suitable emollients can be found in the Cosmetic Bench Reference, pp. 1.19-1.22 (1996). Suitable emollients include polar emollient emulsifiers (such as linear or branched chained polyglycerol esters) and non-polar emollients.

By "polar emollient," as used herein, is meant any emollient emulsifier having at least one polar moiety and wherein the solubility (at 30°C) of the compound in the polar emollient is greater than about 1.5%, greater than about 2%, or greater than about 3%. Suitable polar emollients include, but are not limited to, polyol ester and polyol ethers such as linear or branched chained polyglycerol esters and polyglycerol ethers. Nonlimiting examples of such emollients include polyglyceryl-3-diisosterate, polyglyceryl-2-sesquiisostearate, polyglyceryl-5-distearate, polyglyceryl-10-distearate, polyglyceryl-10-diisostearate, acetylated monoglycerides, glycerol esters, glycerol tricaprylate/caprate, glyceryl ricinoleate, glyceryl isostearate, glyceryl myristate, glyceryl linoleate, polyalkylene glycols such as PEG 600, monoglycerides, 2-monolaurin, sorbitan esters and mixtures thereof.

By "non-polar emollient," as used herein, means any, emollient emulsifier possessing no permanent electric moments. Suitable non-polar emollients include, but are not limited to, esters and linear or branched chained hydrocarbons. Non-limiting examples of such emollients include, but are not limited to, isononyl isononanoate, isopropyl isostearate, octyl hydroxystearate, diisopropyl dimerate, lanolin oil, octyl palmitate, isopropyl palmitate, pariffins, isoparafins, acetylated lanolin, sucrose fatty acid esters, isopropyl myristate, isopropyl stearate, mineral oil, silicone oils, dimethicone, allantoin, isohexadecane, isododecane, petrolatum, and mixtures thereof. The solubility of the compound in polar or non-polar emollients is determined according to methods known in the art.

Oils that act as emollients also impart viscosity, tackiness, and drag properties to cosmetic compositions such as lipstick. Examples of suitable oils include, but are not limited to, caprylic triglycerides; capric triglyceride; isostearic triglyceride; adipic triglyceride; propylene glycol myristyl acetate; lanolin; lanolin oil; polybutene; isopropyl palmitate; isopropyl myristate; isopropyl isostearate; diethyl sebacate; diisopropyl adipate; tocopheryl acetate; tocopheryl linoleate; hexadecyl stearate; ethyl lactate; cetyl oleate; cetyl ricinoleate; oleyl alcohol; hexadecyl alcohol; octyl hyroxystearate; octyl dodecanol; wheat germ oil; hydrogenated vegetable oils; castor oil; petrolatum; modified lanolins; branched-chain hydrocarbons; alcohols and esters; corn oil; cottonseed oil; olive oil; palm kernel oil; rapeseed oil; safflower oil; jojoba oil; evening primrose oil; avocado oil; mineral oil; shea butter; octylpalmitate; maleated soybean oil; glycerol trioctanoate; diisopropyl dimerate; and volatile and non-volatile silicone oils including phenyl trimethicone.

Suitable oils for use herein are acetylglycerides, octanoates, and decanoates of alcohols and polyalcohols, such as those of glycol and glycerol, the ricinoleates of alcohols and polyalcohols such as cetyl ricinoleate, polyglyceryl-3 diisostearate, polyglycerol ethers, polyglyerol esters, caprylic triglycerides, capric triglycerides, isostearic triglyceride, adipic triglyceride, phenyl trimethicone, lanolin oil, polybutene, isopropyl palmitate, isopropyl isostearate, cetyl ricinoleate, octyl dodecanol, oleyl alcohol, hydrogenated vegetable oils, castor oil, modified lanolins, octyl palmitate, lanolin oil, maleated soybean oil, cetyl ricinoleate, glyceryl trioctanoate, diisopropyl dimerate, synthetic lanolin derivatives and branched chain alcohols, sucrose esters of fatty acids, octyl hydroxystearate, and mixtures thereof.

A surfactant may also be added to compositions of the invention, in order to confer beneficial application properties. Surfactants suitable for use are those which can form emulsions and/or association structures. Surfactants suitable for use do not present dermatological or toxicological problems. Anionic surfactants, nonionic surfactants, cationic surfactants, amphoteric surfactants and mixtures thereof are suitable for use. For example, anionic surfactants, nonionic surfactants, cationic surfactants, amphoteric surfactants and mixtures thereof having a Krafft point at or below about ambient temperature are used.

The compositions of this invention may contain one or more materials, herein singly or collectively referred to as a "solidifying agent", that is effective to solidify the particular liquid base materials to be used in a cosmetic composition. As used herein, the term "solidify" refers to the physical and/or chemical alteration of the liquid base material so as to form a solid or semi-solid at ambient conditions, i.e., to form a final composition that has a stable physical structure and can be deposited on the skin under normal use conditions. As is appreciated by those skilled in the art, the selection of the particular solidifying agent for use in the cosmetic compositions will depend upon the particular type of composition desired, i.e., gel or wax-based, the desired rheology, the liquid base material used and the other materials to be used in the composition.

Liposomal formulations may also be useful for the compositions of the present invention. Such compositions can be prepared by combining a composition of the present invention with a phospholipid, such as dipalmitoylphosphatidyl choline, cholesterol and water according to known methods, for example, as described in Mezei et al., J. Pharm. Pharmacol. 34:473-474 (1982), or a modification thereof. Lipids suitable for forming liposomes may be substituted for the phospholipid, as may be lecithin, as well. The liposome preparation is then incorporated into one of the above topical formulations (for example, a gel or an oil-in-water emulsion) in order to produce the liposomal formulation. Other compositions and pharmaceutical uses of topically applied liposomes are described, for example, in Mezei, M. Topics in Pharmaceutical Sciences, Breimer et al. eds., Elsevier Science, New York, N.Y., pp. 345-358 (1985).

Topical compositions of the present invention may also be applied to the oral cavity when incorporated in mouth rinses or mouthwashes, or may be used for ophthalmic treatment incorporated in eyewashes, eyedrops, or eye swabs.

Another manner of administration for the conditions detailed above is oral, using a convenient daily dosage regimen which can be adjusted according to the degree of affliction. For such oral administration, a pharmaceutically acceptable, non-toxic composition is formed by the incorporation of any of the normally employed excipients, such as, for example, mannitol, lactose, starch, magnesium stearate, sodium saccharine, talcum, cellulose, sodium crosscarmellose, glucose, gelatin, sucrose, magnesium carbonate, and the like. Such compositions take the form of solutions, suspensions, tablets, dispersible tablets, pills, capsules, powders, sustained release formulations and the like.

Compositions may take the form of a pill or tablet, and thus the composition may contain, along with the active ingredient, a diluent such as lactose, sucrose, dicalcium phosphate, or the like; a lubricant such as magnesium stearate or the like; and a binder such as starch, gum acacia, polyvinylpyrrolidine, gelatin, cellulose and derivatives thereof, and the like.

In preparing a formulation, it may be necessary to mill the active compound to provide the appropriate particle size prior to combining with the other ingredients. If the active compound is substantially insoluble, it ordinarily is milled to a particle size of less than 200 mesh. If the active compound is substantially water soluble, the particle size is normally adjusted by milling to provide a substantially uniform distribution in the formulation, e.g., about 40 mesh.

Some examples of suitable excipient for oral preparations include lactose, dextrose, sucrose, sorbitol, mannitol, starches, gum acacia, calcium phosphate, alginates, tragacanth, gelatin, calcium silicate, microcrystalline cellulose, polyvinylpyrrolidone, cellulose, water, syrup, and ethyl cellulose. The formulations can additionally include: lubricating agents such as talc, magnesium stearate, and mineral oil; wetting agents; emulsifying and suspending agents; preserving agents such as methyl- and propylhydroxy-benzoates; sweetening agents; and flavoring agents. The compositions of the invention can be formulated so as to provide quick, sustained or delayed release of the active ingredient after administration to the patient by employing procedures known in the art.

The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient.

For preparing solid compositions such as tablets, the principal active ingredient is mixed with a pharmaceutical excipient to form a solid preformulation composition containing a homogeneous mixture of a compound of the present invention. When referring to these preformulation compositions as homogeneous, it is meant that the active ingredient is dispersed evenly throughout the composition so that the composition may be readily subdivided into equally effective unit dosage forms such as tablets, pills and capsules. This solid preformulation is then subdivided into unit dosage forms of the type described above containing from, for example, 0.1 to about 500 mg of the active ingredient of the present invention.

The tablets or pills of the present invention may be coated or otherwise compounded to provide a dosage form affording the advantage of prolonged action. For example, the tablet or pill can include an inner dosage and an outer dosage component, the latter being in the form of an envelope over the former. The two components can be separated by an enteric layer which serves to resist disintegration in the stomach and permit the inner component to pass intact into the duodenum or to be delayed in release. A variety of materials can be used for such enteric layers or coatings, such materials including a number of polymeric acids and mixtures of polymeric acids with such materials as shellac, cetyl alcohol, and cellulose acetate.

Liquid pharmaceutically administrable compositions can, for example, be prepared by dissolving, dispersing, etc. an active compound as defined above and optional pharmaceutical adjuvants in a carrier, such as, for example, water, saline, aqueous dextrose, glycerol, glycols, ethanol, and the like, to thereby form a solution or suspension. If desired, the pharmaceutical composition to be administered may also contain minor amounts of nontoxic auxiliary substances such as wetting agents; emulsifying agents; solubilizing agents; pH buffering agents and the like, for example, sodium acetate, sodium citrate, cyclodextrine derivatives, sorbitan monolaurate, triethanolamine acetate, triethanolamine oleate, etc: Actual methods of preparing such dosage forms are known, or will be apparent, to those skilled in this art; for example, see Remington's Pharmaceutical Sciences, Mack Publishing Company, Easton, Pennsylvania, 15th Edition (1975). The composition or formulation to be administered will, in any event, contain a quantity of the active compound in an amount effective to allevjate the symptoms of the subject being treated.

The liquid forms in which the novel compositions of the present invention may be incorporated for administration orally include aqueous solutions suitably flavored syrups; aqueous or oil suspensions; and flavored emulsions with edible oils such as cottonseed oil, sesame, oil, coconut oil, or peanut oil, as well as elixirs and/or similar pharmaceutical vehicles.

Alternatively, liquid or semi-solid oral formulations may be prepared by dissolving or dispersing the active compound or salt in vegetable oils, glycols, triglycerides, propylene glycol esters (e.g. propylene carbonate) and the like, and encapsulating these solutions or suspensions in hard or soft gelatin capsule shells.

This invention includes compositions associated with pharmaceutically acceptable carriers. In making the compositions of this invention, the active ingredient is usually mixed with an excipient, diluted by an excipient or enclosed within such a carrier which can be in the form of a capsule, sachet, paper or other container. When the excipient serves as a diluent, it can be a solid, semi-solid, or liquid material, which acts as a vehicle, carrier or medium for the active ingredient. Thus, the oral compositions discussed above can be in the form of tablets, pills, powders, lozenges, sachets, cachets, elixirs, suspensions, emulsions, solutions, syrups, aerosols (as a solid or in a liquid medium), ointments containing, for example, up to 10% by weight of the active compound, soft and hard gelatin capsules, suppositories, sterile injectable solutions, and sterile packaged powders.

Parenteral administration can employ the implantation of a slow-release or sustained-release system, such that a constant level of dosage is maintained. The percentage of active compound contained in such parenteral compositions is highly dependent on the specific nature thereof, as well as the activity of the compound and the needs of the subject.

Compositions of the present invention can be used alone or in combination with one or more additional beneficial agent, for example with an anesthetic, an analgesic, an antiinfective, an antibacterial, or an antifungal agent, or mixtures thereof. Some examples of suitable anesthetics that may be added to the compositions of the present invention in order to provide alleviation of pain and itching include, but are not limited to, benzocaine, lidocaine, tetracaine, dyclonine, pramoxine, butamben, camphor, menthol, eucalyptol, thymol, dibucaine, bupivocaine, carbocaine, ropivocaine, procaine, cocaine, novocaine, xylocaine, mepivacaine, benzethonium chloride, anethol, hexetidine, eugenol, caffeine, nicotine, combination of lidocaine and prilocaine, oil of cloves, tea tree oil, lidocaine hydrochloride, dibucaine hydrochloride, tetracaine hydrochloride, tronothane, dyclonine hydrochloride, pramoxine hydrochloride, diperodon, butamben picrate, cyclomethycaine sulfate, cyclomethycaine hydrochloride, dimethisoquin hydrochloride, opoid analgesics such as morphine and its derivatives, and psychoactive drugs including tricyclic antidepressant drugs (TCAs).

Some examples of suitable analgesics that may be added to the compositions of the present invention in order to provide relief from fever, aches and pains that may be associated with the virus, include, but are not limited to, acetaminophen, ibuprofen, aspirin, salicyclamide, trolamine salicylate, methyl salicylate, salicylate salts, N,N-dimethyl aspartic acid, N,N-dimethyl glutamic acid, tripelennamine hydrochloride, hydrocortisone, hydrocortisone acetate and antipyrine.

Some examples of suitable antiinfectives or antibacterials that may also be added to the compositions of the present invention in order to inhibit the spread of infection that may be associated with the virus, include benzalkonium bromide, benzalkonium chloride, chlorhexidine hydrochloride, triclosan, sorbic acid, benzethonium chloride, methyl benzethonium chloride, alcohol, cetyl pyridinium chloride, chloroxylenol, hexachlorophene, and chlorhexidine.

Examples of topical antifungals that may be added to the compositions of the present invention in order to control fungal growth that may be associated with the sores, include, but are not limited to, haloprogin, ciclopirox, flucytosine, miconazole, econazole, clotrimazole, fluconazole, oxiconazole, sulconazole, metronidazole, itraconazole, ketoconazole, butaconazole, terconazole, nystatin, povidone-iodine, tolnaftate, terbinafine hydrochloride, micatin, nystatin, amphorericin B, griseofulvin, benzoic acid, salicylic acid, mercuric oxide, resorcinol, triacetin, undecylenic acid and its calcium, copper and zinc salts.

### EXAMPLES

The following preparations and examples are given to enable those skilled in the art to more clearly understand and to practice the present invention. They should not be considered as limiting the scope of the invention, but merely as being illustrative and representative thereof.

### Example 1

### HSV-1 Assay

Vero cells, (ATCC CCL-81) are pregrown in 96-well tissue culture plates using Dulbecco's Modified Eagle's Medium (DMEM) supplemented with 10% heat-inactivated fetal bovine serum (FBS), L-Glutamine, penicillin, and streptomycin.

To each of the replicate cell cultures is added 50 µL of the test article solution and 50 µL of virus suspension (ATCC VR-260). The multiplicity of infection used is about 0.05 plaque-forming unit (PFU) per cell. Cell controls containing medium alone, virus-infected controls containing medium and virus, drug cytotoxicity controls containing medium and each drug concentration, reagent controls containing culture medium only (no cells), and the test article colorimetric controls containing the test article and medium (no cells) are run simultaneously with the test samples. The plates are incubated at 37°C in a humidified atmosphere containing 5% CO₂ until maximum CPE (cytopathic effect) is observed in the untreated virus control cultures (Day 5).

CPE inhibition is determined by a dye (MTS) uptake procedure (Promega's Cell Titer Aqueous One Solution). This method measures cell viability and is based on the reduction of the tetrazolium-based MTS by mitochondrial enzymes of viable host cells to MTS formazan. MTS (10 µl) is added to each of the plate wells. The plates are incubated at 37°C for 4 hours. The purple color of the MTS formazan is then measured spectrophotometrically at 490/650 nm. The optical density (OD) value of each culture is a function of the amount of formazan produced which is proportional to the number of viable cells.

The percent of CPE (cytopathic effect) reduction of the virus-infected wells (antiviral efficacy) and the percent cell viability of uninfected drug control wells (cytotoxicity) are calculated. IC₅₀ (inhibitory concentration at which the compound provides 50% CPE reduction) and TC₅₀ (cytotoxic concentration at which the compound causes the death of 50% of the cells) are calculated using a computer program.

Compounds of the present invention when tested as described above showed reduction of viral replication.

| Compound | Structure | IC₅₀ µM |
|---|---|---|
| 2',4'-Dihydroxy-3,4-dimethoxychalcone | 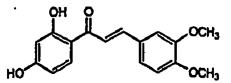 | 8.36 |
| 2',3,4,4'-Tetrahydroxychalcone | 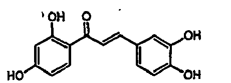 | 26.6 |
| 2',4'-Dihydroxy-4-methoxychalcone | 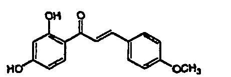 | 11.1 |
| (Z)-2-(3,4-Dihydroxybenzylidene)-6-hydroxybenzofuran-3(2H)-one | 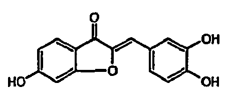 | >100 |

### Example 2

### Inflammation assay- Cell E-Selectin Assay

Endothelial-Leukocyte Adhesion Molecule (ELAM), also known as E-selectin, was expressed on the surface of endothelial cells. In this assay, lipopolysaccharide (LPS) and IL-1β were used to stimulate the expression of E-selectin, test agents were tested for their abilities to reduce this expression, in accordance with studies showing that reduction of leukocyte adhesion to endothelial cell surface was associated with decreased cellular damage (e.g., Takada, M., Et al., Transplantation 64: 1520-25, 1997; Steinberg, J.B., et al., J. Heart Lung Trans. 13:306-313, 1994).

Endothelial cells may be selected from any of a number of sources and cultured according to methods known in the art, including, for example, coronary artery endothelial cells, human brain microvascular endothelial cells (HBMEC; Hess, D.C., et al., Neurosci. Lett. 213(1): 37-40, 1996), or lung endothelial cells. Cells were conveniently cultured in 96-well plates. Cells were stimulated by adding a solution to each well containing 10 µg/mL LPS and 100 µg/ml IL-1β for 6 hours in the presence of test agent (specific concentrations and time may be adjusted depending on the cell type). Treatment buffer was removed and replaced with pre-warmed Fixing Solution® (100 µL/well) for 25 minutes at room temperature. Cells were then washed 3X, then incubated with Blocking Buffer (PBS + 2% FBS) for 25 minutes at room temperature. Blocking Buffer containing Monoclonal E-Selectin Antibody (1:750, Sigma Catalog #S-9555) was added to each well. Plates were sealed and stored at 4°C overnight. Plates were washed 4X with 160 µL Blocking Buffer per well. Second Antibody-HRP diluted 1:5000 in Blocking Buffer was then added (100 µL/well), and plates were incubated at room temperature (protected from light) for two hours. Plates were then washed 4X with Blocking Buffer before addition of 100 µL of ABTS Substrate solution at room temperature (Zymed, Catalog #00-2024). Wells were allowed to develop for 35 minutes, before measurement at 402 nm in a Fluoroskan® Reader with shake program for 10 seconds. Positive results were recorded as a decrease in E-selectin concentration in tested wells, as compared to control wells.

Certain compounds of this invention when tested as described above showed some activity at an EC₅₀ of 10µM or less.

### Example 3

### Inflammation assay- IL-6 Assay

The purpose of this assay is to measure IL-6 from a macrophage cell using an ELISA technique. This assay measures the release of IL-6 from a rat macrophage cell line (NR8383) following an inflammatory challenge with LPS and the ability of test articles to inhibit this activation and release. IL-6 is measured by a rat IL-6 ELISA (IL-6 ELISA kit is available by Pierce/Endogen #ER2-IL6) and cell toxicity is determined using Cell Tracker.

### Materials and Equipments:

### MATERIALS FOR CELL PREPARATION AND EXPERIMENT

- NR8383 cell line (ATCC #CRL-2192)
- Kaighn's F12 media (Gibco #211127-022)
- FBS (Hyclone SH30070.03)
- Penicillin/Streptomycin, 100X (Gibco 15140-122).
- LPS (Sigma L2537) (stock at 5mg/ml in DMSO, aliquot and store in the freezer)
- Cell Tracker Green (Molecular Probe # C2925 1 mg or #C7025 20*50)
- Cell tracker MW=465 (10mM is 1 mg in 215 uL DMSO aliquot and store in the freezer)
- HBSS buffer (see Appendix 10.1, 10,2)
- 96-well black clear bottom plate (VWR # 29442-152)
- 96-well deep well mother plate, DyNA Block 1000 (VWR # 40002-008)

### EXPERIMENTAL PREPARATION AND PROCEDURE:

### SEEDING NR8383 INTO 96-WELL PLATES:

96-Well clear bottom black plates were coated with 5% FBS in Kaighn's media (470ml of media + 25 ml of FBS + 5ml of pen/step) (100 µL per well) and incubated for 30 minutes at 37°C then the 5% media solution was removed by aspiration. The NR8383 cells were seeded in 15%-FBS Kaighn's media and grown at 37°C for 24 hours prior to use in the IL-6 assay.

### LPS ACTIVATION AND PLATE SET-UP FOR TEST ARTICLE:

24 hours after seeding, the cells were stimulated with LPS at a final concentration of 10ug/mL in the NR8383 growth media. The NR8383 cell plate was retrieved from the incubator, and the plates were prepared as follows: for each 96-well plate, 30 mL of NR8383 growth media was warmed up, and 3 mL were removed for the negative control. To the remaining 27 mL media were add 54 µL of 5 mg/mL LPS and and 3 mL were removed for the positive control. In 15 mL Falcon tubes, 2 mL of the LPS-media solution was dispensed. The test articles were added; at their highest concentration to their respective tubes, the old media was removed and 200 µL from each well from the prepared mother dish was added to each respective well of the cell plate. The cell plates were incubated for another 24 hours in the 37°C incubator.

The cell plates were ready for cell viability measurement using Cell Tracker, and the amount of IL-6 in the supernatants was measured by the IL-6 ELISA run by a kit available by Pierce/Endogen #ER2-IL6.
Compounds of the present invention were tested for their ability to reduce inflammation in this model.

### Example 4

### High Glutamate-Induced Oxidative Stress Assay (HGOS)

This procedure was used to induce high glutamate-induced oxidative, stress (HGOS) in a dopaminergic neuronal cell line. Using this assay the potency and efficacy of test articles against HGOS neuronal cell injury and cell death was established in a high throughput manner.

### Materials

- Dopaminergic neuronal cell lines
- DMEM-No Glucose (Life Technologies Cat # 11966-025)
- L-glutamine (Life Technologies Cat # 25030-081)
- L-glutamic acid, monosodium salt (Sigma Cat # G5889)
- D-glucose (Sigma Cat # G-6151)
- 10x HBSS buffer(pH 7.4) (950 mL Pyrogen-free water, 2.44g/L MgCl₂.6H₂0, 3.73g/L KCl, 59.58g/L Hepes, 58.44g/L NaCl, 1.36g/L KH₂PO₄, 1.91g/L CaCl₂ .2H₂O and pH to 4.5 with HCl)
- Cell Tracker Green fluorescent dye (Molecular Probes, Cat # 2925). Prepare a 5 µM solution in pre-warmed HBSS just prior to use.
- Sterile 96-well plates precoated with poly-D-lysine (Corning Catalog # 3665)
- 96-well deep well mother plate, DyNA Block 1000 (VW R Catalog # 40002-008)

### Neuronal Cells

The cells were seeded into 96-well plates at a density of 2000 per well and left to grow for 72 hours in a 33°C incubator with 5% CO₂ in air atmosphere. The passage number of the cells for each assay experiment were no later than p=11 in order to minimize experimental variation.

### Compound Preparation in Deep-well Mother Plates

VWRBrand DyNA Block 1000, deep well mother plates (VWR Cat. # 40002-008) were used for the preparation of the test compounds.

All compounds were dissolved in DMEM-No Glu containing 1 mM glucose, 30 mM glutamate and 1x Pen/Strep. DMEM-No Glu with 1 mM glucose and 1x P/S was used as the negative control, DMEM-No Glucose with 1 mM glucose, 100 M glutamate was used as a positive control and 100 µM Glutathione was added to the positive control as a standard. All of the procedures for this involving the making and dilution of compounds were performed using aseptic conditions and with minimal light.

### Cell Preparation

The plates were removed from the incubator and examined under the microscope for morphological appearance and density. Using an aseptic technique and an 8-channel aspirator the media was carefully removed from the cells and replaced with 200 µl of 1x HBSS. This was done as quickly as possible to prevent the cells drying out. The plates were then placed in the humidified 37°C incubators of the Biomek 2000 Side Loader. Four plates were washed at a time so as to minimize the time that the cells were sitting in 1 x HBSS prior to addition of the compound test solution.

### Experimental Setup

The Beckman Biomek workstations were used to load the compounds and controls from the mother plates onto the cell plates that were prewashed with HBSS under sterile conditions. The plates were incubated in the upper HTS incubator at 37°C in 5% CO₂ for exactly 16 h. The following day, using the Beckman Biomek workstations, the plates were removed from the incubator. Using Cell Tracker Addition, the compounds were removed from the plates, washed once with 200 µM of pre-warmed 1x HBSS and then 100 µL of 5 µM Cell Tracker Green was added to each well. The plates were incubated at 37°C for 30 min to allow the dye to enter the cell and be cleaved by the esterases. After washing the cells twice with prewarmed 1 x HBSS, the plates were read with the 485 excitation; 538 emission filter pair on a Fluoroskan.

Certain compounds of the present invention were active and exhibited protection against HGOS cell injury and cell death.

## Claims

1. The use of a compound of Formula IA: 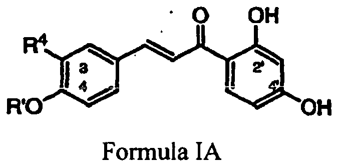 wherein,
R⁴ is hydrogen or -OR'; and
R' is hydrogen or C₁-C₆-alkyl;
including single stereoisomers, mixtures of stereoisomers, and pharmaceutically acceptable salts thereof,
for the manufacture of a medicament for the treatment of a cold sore or for controlling viral growth and replication resulting from herpes simplex virus.

2. A compound of Formula IA: 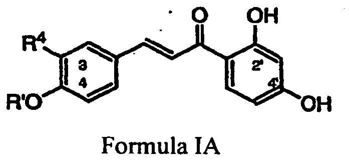 wherein,
R⁴ is hydrogen or -OR'; and
R' is hydrogen or C₁-C₆-alkyl;
including single stereoisomers, mixtures of stereoisomers, and pharmaceutically acceptable salts thereof,
for use in a method of treating a cold sore or for controlling viral growth and replication resulting from herpes simplex virus.

3. A composition comprising the compound of claim 2 for use in a method of treating a cold sore, or for controlling viral growth and replication resulting from herpes simplex virus.

4. The use, compound for use or composition for use of any of claims 1 to 3, wherein the composition is administered topically.

5. The use, compound for use or composition for use of any of claims 1 to 3, wherein the composition is administered orally.

6. The use, compound for use or composition for use of any of claims 1 to 3, wherein the composition further comprises at least one agent selected from one of the following groups:
(i) a skin protectant active ingredient selected from allantoin, aluminum hydroxide gel, calamine, cocoa butter, cod liver oil, colloidal oatmeal, dimethicone, glycerin, hard fat, kaolin, lanolin, mineral oil, petrolatum, sodium bicarbonate, topical starch, white petrolatum, zinc acetate, and/or zinc oxide, and
(ii) an external analgesic, anesthetic or antipruritic ingredient selected from benzocaine, butamaben picrate, dibucaine, dibucaine hydrochloride, dimethiosoquin hydrochloride, dyclonine hydrochloride, lidocaine, lidocaine hydrochloride, pramoxine hydrochloride, tetracaine, tetracaine hydrochloride, benzyl alcohol, camphor, camphorated metacresol, juniper tar, menthol, phenol, phenolate sodium resorcinol, tripelennamine hydrochloride, aspirin, hydrocortisone, hydrocortisone acetate and/or diphenydramine hydrochloride.

7. The use, compound for use or composition for use of any of claims 1 to 3, wherein the composition further comprises at least one other agent selected from the group consisting of anti-microbial agents, other antiviral agents, antifungal agents, antioxidants, buffering agents, sunscreens, cosmetic agents, fragrances, lubricants, moisturizers, drying agents, and thickening agents.

8. The use, compound for use or composition for use of any of claims 1 to 3, wherein the compound is selected from 2',4'-dihydroxy-3,4-dimethoxychalcone, 2',4'-dihydroxy-4-methoxychalcone, 2',3,4,4'-tetrahydroxychalcone, 2',4',4-trihydroxychalcone, 2',4,4'-trihydroxy-3-methoxychalcone, and 2',3,4'-trihydroxy-4-methoxychalcone including single stereoisomers, mixtures of stereoisomers, and pharmaceutically acceptable salts thereof.

9. The use, compound for use or composition for use of any of claims 1 to 3, wherein the compound is 2',4'-dihydroxy-3,4-dimethoxychalcone including single stereoisomers, mixtures of stereoisomers, and pharmaceutically acceptable salts thereof.

10. The use, compound for use or composition for use of any of claims 1 to 3, wherein the compound is 2',3,4,4'-tetrahydroxychalcone, including single stereoisomers, mixtures of stereoisomers, and pharmaceutically acceptable salts thereof.

11. The use, compound for use or composition for use of any of claims 1 to 3, wherein the compound is 2',4'-dihydroxy-4-methoxychalcone including single stereoisomers, mixtures of stereoisomers, and pharmaceutically acceptable salts thereof.

12. The use, compound for use or composition for use of any of claims 1 to 3, wherein the viral infection results from herpes simplex virus-1.

## Patentansprüche

1. Verwendung einer Verbindung der Formel IA: 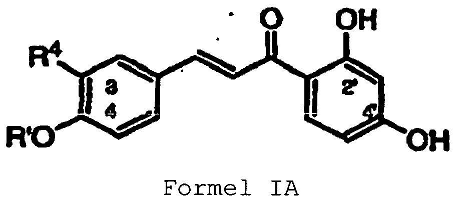 worin
R⁴ für Wasserstoff oder -OR' steht und
R' für Wasserstoff oder C₁-C₆-Alkyl steht;
einschließlich von einzelnen Stereoisomeren, Gemischen von Stereoisomeren und pharmazeutisch unbedenklichen Salzen davon
zur Herstellung eines Medikaments zur Behandlung von Herpes simplex oder zur Bekämpfung von Virenwachstum und -replikation als Resultat des Herpes-simplex-Virus.

2. Verbindung der Formel IA: 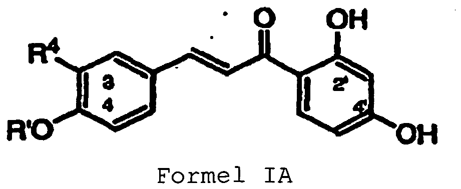 worin
R⁴ für Wasserstoff oder -OR' steht und
R' für Wasserstoff oder C₁-C₆-Alkyl steht;
einschließlich von einzelnen Stereoisomeren, Gemischen von Stereoisomeren und pharmazeutisch unbedenklichen Salzen davon
zur Verwendung bei einem Verfahren zur Behandlung von Herpes simplex oder zur Bekämpfung von Virenwachstum und -replikation als Resultat des Herpes-simplex-Virus.

3. Zusammensetzung, umfassend die Verbindung nach Anspruch 2, zur Verwendung bei einem Verfahren zur Behandlung von Herpes simplex oder zur Bekämpfung von Virenwachstum und -replikation als Resultat des Herpes-simplex-Virus.

4. Verwendung, Verbindung zur Verwendung oder Zusammensetzung zur Verwendung nach einem der Ansprüche 1 bis 3, wobei die Zusammensetzung topisch verabreicht wird.

5. Verwendung, Verbindung zur Verwendung oder Zusammensetzung zur Verwendung nach einem der Ansprüche 1 bis 3, wobei die Zusammensetzung oral verabreicht wird.

6. Verwendung, Verbindung zur Verwendung oder Zusammensetzung zur Verwendung nach einem der Ansprüche 1 bis 3, wobei die Zusammensetzung ferner mindestens ein Mittel umfasst, das aus einer der folgenden Gruppen ausgewählt ist:
(i) einem hautschützenden Wirkstoff, ausgewählt aus Allantoin, Aluminiumhydroxidgel, Calamin, Kakaobutter, Dorschleberöl, Kolloidhaferflocken, Dimethicon, Glycerin, Hartfett, Kaolin, Lanolin, Mineralöl, Petrolatum, Natriumbicarbonat, topischer Stärke, weißem Petrolatum, Zinkacetat und/oder Zinkoxid, und
(ii) einem externen Analgetikum, Anästhetikum oder Antipruritikum, ausgewählt aus Benzocain, Butamabenpikrat, Dibucain, Dibucainhydrochlorid, Dimethiosoquinhydrochlorid, Dycloninhydrochlorid, Lidocain, Lidocainhydrochlorid, Pramoxinhydrochlorid, Tetracain, Tetracainhydrochlorid, Benzylalkohol, Campher, Campher-Metakresol, Wacholderteer, Menthol, Phenol, Natriumphenolat, Resorcin, Tripelennaminhydrochlorid, Aspirin, Hydrocortison, Hydrocortisonacetat und/oder Diphenhydraminhydrochlorid.

7. Verwendung, Verbindung zur Verwendung oder Zusammensetzung zur Verwendung nach einem der Ansprüche 1 bis 3, wobei die Zusammensetzung ferner mindestens ein anderes Mittel umfasst, das aus der Gruppe bestehend aus antimikrobiellen Mitteln, anderen antiviralen Mitteln, Antimykotika, Antioxidantien, Puffersubstanzen, Sonnenschutzmitteln, kosmetischen Mitteln, Duftstoffen, Gleitmitteln, Feuchtigkeitsmitteln, Trockenmitteln und Verdickungsmitteln ausgewählt ist.

8. Verwendung, Verbindung zur Verwendung oder Zusammensetzung zur Verwendung nach einem der Ansprüche 1 bis 3, wobei die Verbindung aus 2',4'-Dihydroxy-3,4-dimethoxychalkon, 2',4'-Dihydroxy-4-methoxychalkon, 2',3,4,4'-Tetrahydroxychalkon, 2',4',4-Trihydroxychalkon, 2',4,4'-Trihydroxy-3-methoxychalkon und 2',3,4'-Trihydroxy-4-methoxychalkon einschließlich von einzelnen Stereoisomeren, Gemischen von Stereoisomeren und pharmazeutisch unbedenklichen Salzen davon ausgewählt ist.

9. Verwendung, Verbindung zur Verwendung oder Zusammensetzung zur Verwendung nach einem der Ansprüche 1 bis 3, wobei es sich bei der Verbindung um 2',4'-Dihydroxy-3,4-dimethoxychalkon einschließlich von einzelnen Stereoisomeren, Gemischen von Stereoisomeren und pharmazeutisch unbedenklichen Salzen davon handelt.

10. Verwendung, Verbindung zur Verwendung oder Zusammensetzung zur Verwendung nach einem der Ansprüche 1 bis 3, wobei es sich bei der Verbindung um 2',3,4,4'-Tetrahydroxychalkon einschließlich von einzelnen Stereoisomeren, Gemischen von Stereoisomeren und pharmazeutisch unbedenklichen Salzen davon handelt.

11. Verwendung, Verbindung zur Verwendung oder Zusammensetzung zur Verwendung nach einem der Ansprüche 1 bis 3, wobei es sich bei der Verbindung um 2',4'-Dihydroxy-4-methoxychalkon einschließlich von einzelnen Stereoisomeren, Gemischen von Stereoisomeren und pharmazeutisch unbedenklichen Salzen davon handelt.

12. Verwendung, Verbindung zur Verwendung oder Zusammensetzung zur Verwendung nach einem der Ansprüche 1 bis 3, wobei die Virusinfektion aus dem Herpes-simplex-Virus 1 resultiert.

## Revendications

1. Utilisation d'un composé de Formule IA : 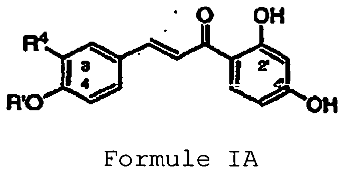 dans laquelle
R⁴ est hydrogène ou -OR' ; et
R' est hydrogène ou C₁-C₆-alkyle ;
y compris les stéréoisomères uniques, les mélanges de stéréoisomères et les sels pharmaceutiquement acceptables de celui-ci,
pour la fabrication d'un médicament destiné à traiter un bouton de fièvre ou à contrôler la croissance et la réplication virales provenant du virus Herpes simplex.

2. Composé de formule IA : 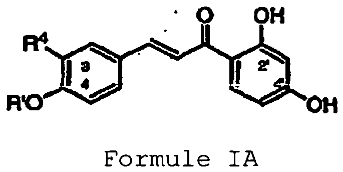 dans laquelle
R⁴ est hydrogène ou -OR' ; et
R' est hydrogène ou C₁-C₆-alkyle ;
y compris les stéréoisomères uniques, les mélanges de stéréoisomères et les sels pharmaceutiquement acceptables de celui-ci,
destiné à être utilisé dans une méthode pour traiter un bouton de fièvre ou pour contrôler la croissance et la réplication virales provenant du virus Herpes simplex.

3. Composition comprenant le composé selon la revendication 2, destinée à être utilisée dans une méthode pour traiter un bouton de fièvre ou pour contrôler la croissance et la réplication virales provenant du virus Herpes simplex.

4. Utilisation, composé à utiliser ou composition à utiliser selon l'une quelconque des revendications 1 à 3, **caractérisés en ce que** la composition est administrée par voie topique.

5. Utilisation, composé à utiliser ou composition à utiliser selon l'une quelconque des revendications 1 à 3, **caractérisés en ce que** la composition est administrée par voie orale.

6. Utilisation, composé à utiliser ou composition à utiliser selon l'une quelconque des revendications 1 à 3, **caractérisés en ce que** la composition comprend en outre au moins un agent choisi dans l'un des groupes suivants :
(i) un principe actif protecteur de la peau, choisi parmi l'allantoïne, le gel d'hydroxyde d'aluminium, la calamine, le beurre de cacao, l'huile de foie de morue, le gruau d'avoine, la diméthicone, la glycérine, la graisse solide, le kaolin, la lanoline, l'huile minérale, le pétrolatum, le bicarbonate de sodium, l'amidon topique, le pétrolatum blanc, l'acétate de zinc, et/ou l'oxyde de zinc, et
(ii) un ingrédient analgésique, anesthétique ou antiprurigineux externe choisi parmi la benzocaïne, le picrate de butamabène, la dibucaïne, le chlorhydrate de dibucaïne, le chlorhydrate de diméthiosoquine, le chlorhydrate de dyclonine, la lidocaïne, le chlorhydrate de lidocaïne, le chlorhydrate de pramoxine, la tétracaïne, le chlorhydrate de tétracaïne, l'alcool benzylique, le camphre, le métacrésol camphré, l'huile de cade, le menthol, le phénol, le phénolate sodique, le resorcinol, le chlorhydrate de tripélennamine, l'aspirine, l'hydrocortisone, l'acétate d'hydrocortisone et/ou le chlorhydrate de diphénydramine.

7. Utilisation, composé à utiliser ou composition à utiliser selon l'une quelconque des revendications 1 à 3, **caractérisés en ce que** la composition comprend en outre au moins un autre agent choisi dans le groupe constitué par les agents antimicrobiens, d'autres agents antiviraux, les agents antifongiques, les antioxydants, les agents tampons, les filtres solaires, les agents cosmétiques, les parfums, les lubrifiants, les hydratants, les agents de séchage et les agents épaississants.

8. Utilisation, composé à utiliser ou composition à utiliser selon l'une quelconque des revendications 1 à 3, **caractérisés en ce que** le composé est choisi parmi la 2',4'-dihydroxy-3,4-diméthoxychalcone, la 2'4'-dihydroxy-4-méthoxychalcone, la 2',3,4,4'-tétrahydroxychalcone, la 2',4',4-trihydroxychalcone, la 2',4,4'-trihydroxy-3-méthoxychalcone, et la 2',3,4'-trihydroxy-4-méthoxychalcone y compris les stéréoisomères uniques, les mélanges de stéréoisomères et les sels pharmaceutiquement acceptables de celles-ci.

9. Utilisation, composé à utiliser ou composition à utiliser selon l'une quelconque des revendications 1 à 3, **caractérisés en ce que** le composé est la 2',4'-dihydroxy-3,4-diméthoxychalcone y compris les stéréoisomères uniques, les mélanges de stéréoisomères et les sels pharmaceutiquement acceptables de celle-ci.

10. Utilisation, composé à utiliser ou composition à utiliser selon l'une quelconque des revendications 1 à 3, **caractérisés en ce que** le composé est la 2',3,4,4'-tétrahydroxychalcone y compris les stéréoisomères uniques, les mélanges de stéréoisomères et les sels pharmaceutiquement acceptables de celle-ci.

11. Utilisation, composé à utiliser ou composition à utiliser selon l'une quelconque des revendications 1 à 3, **caractérisés en ce que** le composé est la 2',4'-dihydroxy-4-méthoxychalcone y compris les stéréoisomères uniques, les mélanges de stéréoisomères et les sels pharmaceutiquement acceptables de celle-ci.

12. Utilisation, composé à utiliser ou composition à utiliser selon l'une quelconque des revendications 1 à 3, **caractérisés en ce que** l'infection virale provient du virus Herpes simplex 1.
